# EUROPEAN PATENT SPECIFICATION

(11) **EP 1 885 714 B1**
(45) Date of publication and mention of the grant of the patent: **15.01.2014**
(21) Application number: 06753776.1
(22) Date of filing: 22.05.2006
(51) Int. Cl.: C07D 403/10

(54) **PROCESS FOR THE PREPARATION OF 2-ALKYL-1-((2'-SUBSTITUTED-BIPHENYL-4-YL)METHYL)-IMIDAZOLE, DIHYDROIMIDAZOLE OR BENZIMIDAZOLE DERIVATIVES**
VERFAHREN ZUR HERSTELLUNG VON 2-ALKYL-1-((2'-SUBSTITUIERTEN-BIPHENYL-4-YL)METHYL)-IMIDAZOL, -DIHYDROIMIDAZOL ODER -BENZIMIDAZOL-DERIVATEN
PROCEDE DE PREPARATION DE DERIVES DE 2-ALKYL-1-((2'-SUBSTITUE-BIPHENYL-4-YL)METHYL)-IMIDAZOLE, DIHYDROIMIDAZOLE OU BENZIMIDAZOLE

(30) Priority: 24.05.2005 SI 200500155
(43) Date of publication of application: 13.02.2008
(73) Proprietor: LEK Pharmaceuticals d.d., 1526 Ljubljana (SI)
(72) Inventor: SELIC, Lovro, 3000 Celje (SI)
(74) Representative: Oser, Andreas
(86) International application number: PCT/EP2006/004843
(87) International publication number: WO 2006/125592

(56) References cited:
- WO-A2-2004/065383
- Dennis G. Hall: "Boronic Acids", 2005, Wiley ISBN: 3-527-30991-8 pages 68-71,

## Description

### Field of the Invention

The invention belongs to the field of organic chemistry and relates to a novel synthetic process for the preparation of 2-alkyl-1-((2'-substituted-biphenyl-4-yl)methyl)- imidazole, dihydroimidazole or benzimidazole derivatives such as 2-butyl-3-[[2'-[1-(triphenylmethyl)-1*H*-tetrazol-5-yl][1,1'-biphenyl]-4-yl]methyl]-1,3-diazaspiro[4.4]non-1-en-4-one, a key intermediate in the synthesis of irbesartan, or 4'-[(1,4'-dimethyl-2'-propyl-[2,6'-bi-1*H*-benzimidazol]-1'-yl)methyl]-[1,1'-biphenyl]-2-carboxylic acid, telmisartan.

### Background of the Invention

Irbesartan and telmisartan together with other angiotensin II antagonists share a common structure consisting of 4,1'-disubstituted biphenyl where one substituent is preferably tetrazole or carboxy group and the other an alkyl substituent which is further substituted, preferably by at least one nitrogen atom containing heterocycle. The preferred angiotensin II antagonists in accordance with our invention are irbesartan, telmisartan, losartan, candesartan, olmesartan and valsartan and their derivatives. The most preferred are irbesartan and telmisartan.

2-Butyl-3-[[2'-[1-(triphenylmethyl)-1*H*-tetrazol-5-yl][1,1'-biphenyl]-4-yl]methyl]-1,3-diazaspiro[4.4]non-1-en-4-one (**I**) is a key intermediate for the synthesis of irbesartan, (2-butyl-3-[[2'-(1*H*-tetrazol-5-yl)[1,1'-biphenyl]-4-yl]methyl]-1,3-diazaspiro[4.4]non-1-en-4-one). Irbesartan is synthesized from compound with formula I, by removal of trityl group.

The synthesis of irbesartan is described, among other compounds, in US 5,270,317 and 5,559,233. The synthesis therein disclosed proceeds by coupling the 2-butyl-1,3-diazaspiro[4.4]non-1-en-4-one with 4'-(bromomethyl)biphenyl-2-carbonitrile, followed by addition of azide to carbonitrile group or reacting the 1-aminocyclopentanecarboxamide with 4'-(aminomethyl)biphenyl-2-carbonitrile and valeric acid ortho-ester, followed by addition of azide.

WO 2004/072964 describes preparation of trityl irbesartan from ethyl 1-aminocyclopentanecarboxylate, (2'-(1-trityl-1*H-*tetrazol-5-yl)biphenyl-4-yl)methanamine and ethyl valerimidate methanesulfonic acid salt.

WO 2004/007482 describes process of making trityl irbesartan from 2-butyl-1,3-diazaspiro[4.4]non-1-en-4-one hydrochloride with 5-(4'-(bromomethyl)biphenyl-2-yl)-1-trityl-1*H*-tetrazole, in the two phase system, using phase transfer catalyst.

WO 2004/065383 disclosed the process of making trityl irbesartan from 2-butyl-3-(4'-bromophenyl)-1,3-diazaspiro[4.4]non-1-ene-4-one with 2-(1-trityl-1*H*-tetrazol-5-yl)phenylboronic acid, using conditions of Suzuki reaction, in the two phase system, in the presence of phase transfer catalyst.

EP 502314 disclosed the process of making telmisartan, among others, from (2'-carboxy-biphenyl-4-yl)methyl derivatives with nucleophilic leaving group and corresponding benzimidazole.

Suzuki coupling reaction has been used for building biphenylic part of the angiotensin II antagonist to which subsequently a heterocyclic part is constructed. This reaction usually needs an efficient catalyst, mainly a palladium catalyst. For industrial application there is a need for not only a safe, robust scalable process, but also for a cheap procedure. Palladium could play an expensive role in the synthetic scheme as a whole, so it is more suitable if enters in a later part of the synthetic pathway because fewer steps to the end of synthesis may mean less opportunity of losses due to incomplete reactions or isolations. Synthetic schemes like US 5,270,317, US 5,559,233, WO 2004/007482 and EP 502314, plan to initially build biphenylic part and subsequently condense it to the heterocyclic part. Thus the catalyst is used in the earlier step. Process in WO 2004/065383 builds biphenylic part into the imidazole structure *per partes* using a catalyst in the later step, but there is still a need for making boronic acid part in a less complicate species then on the phenyltetrazole part.

### Disclosure of the Invention

The invention in one aspect represents a process for reaction of a compound with formula: 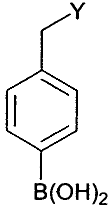 where Y represents leaving group, suitable for substitution with amino group, and is selected from the groups consisting of halogens, sulfonate esters; phosphate of phosphite esters, and chlorosulfites, with optionally substituted imidazole derivative of formula 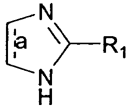 where a can be either single or double bond and said imidazole is selected from any one of those of formulas, 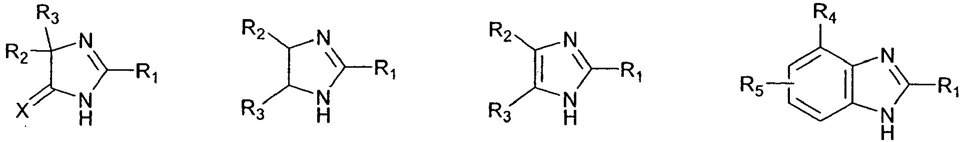 in which:
R₁ is a hydrogen, a C₁-C₆ alkyl which is unsubstituted or substituted by one or more halogen atoms, a C₂-C₆ alkenyl, a C₃-C₇ cycloalkyl, a phenyl, a phenylalkyl in which the alkyl is C₁-C₃, or a phenylalkenyl in which the alkenyl is C₂-C₃, said phenyl groups being substituted or polysubstituted by a halogen atom, a C₁-C₄ alkyl, a C₁-C₄ halogenoalkyl, C₁-C₄ polyhalogenoalkyl, a hydroxyl or a C₁-C₄ alkoxy group,
R₂ and R₃ are same or different and are each independently hydrogen or a group selected from halogen atoms, a C₁-C₆ alkyl which is unsubstituted or substituted by one or more hydroxyl groups or halogen atoms, a C₃-C₇ cycloalkyl, a C₁-C₄ alkoxy, a hydroxyl, an amino, an amino alkyl, a carboxyl, carboxyaldehide, an alkoxycarbonyl in which the alkoxy is C₁-C₄, a cyano, a tetrazolyl,
or R₂ or R₃ form a group of formula -(CH₂)ₙ-O-CO-R₆ where n is an integer between 2 and 6; and R₆ is selected from group consisted of a C₁-C₆ alkyl which is unsubstituted or substituted by one or more halogen, nitro, cyano, hydroxyl or alkoxy group; phenyl which may be unsubstituted or substituted with (nitrooxy)methyl, alkoxymethyl, or halogen atom,
or R₂ and R₃ together form a group of the formula -(CH₂)ₙ-, where n is an integer between 2 and 11,
X is an oxygen or sulphur atom,
R₄ is hydrogen, or a group selected from halogen atoms, a C₁-C₆ alkyl which is unsubstituted or substituted by one or more halogen atoms, a C₃-C₇ cycloalkyl, a C₁-C₄ alkoxy,
R₅ represents a carboxylic group, benzimidazol-2-yl or 4,5,6,7-tetrahydrobenzimidazol-2-yl group, optionally substituted in the 1-position by C₁-C₆ alkyl or a cycloalkyl group, whilst the phenyl nucleus of one of the abovementioned benzimidazole groups may additionally be substituted by a fluorine atom or by methyl or trifluoromethyl group,
or their corresponding hydrohalides
where a compounds presented with any of the formulae 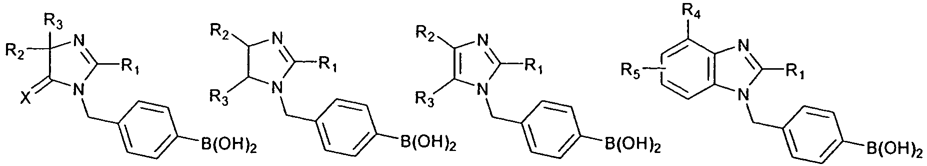 are prepared.

In another aspect the invention is embodied in new compounds: 4-[(2-butyl-4-oxo-1,3-diazaspiro[4.4]non-1-en-3-yl)methyl]phenylboronic acid, which can be used as an intermediate in the process for preparing irbesartan; 1-(4-boronobenzyl)-4-(2-hydroxypropan-2-yl)-2-propyl-1*H*-imidazole-5-carboxylic acid which can be used as an intermediate in the process for preparing olmesartan; 4-((2-butyl-4-chloro-5-(hydroxymethyl)-1*H*-imidazol-1-yl)methyl)phenylboronic acid and 4-((2-butyl-4-chloro-5-formyl-1*H*-imidazol-1-yl)methyl)phenylboronic acid, which can be used as intermediates in the process for preparing losartan; 3-(4-boronobenzyl)-2-ethoxy-3*H*-benzo[d]imidazole-4-carboxylic acid which can be used as an intermediate in the process for preparing candesartan and (*S*)-2-(*N*-(4-boronobenzyl)pentanamido)-3-methylbutanoic acid which can be used as an intermediate in the process for preparing valsartan. The aspects of the invention are also derivatives, preferably salts and protected forms of above compounds, which are preferably esters thereof.

In yet another aspect the invention provides for a use of 4-[(2-butyl-4-oxo-1,3-diazaspiro[4.4]non-1-en-3-yl)methyl]phenylboronic acid or 1-(4-boronobenzyl)-4-(2-hydroxypropan-2-yl)-2-propyl-1*H*-imidazole-5-carboxylic acid or 4-((2-butyl-4-chloro-5-(hydroxymethyl)-1*H*-imidazol-1-yl)methyl)phenylboronic acid or 4-((2-butyl-4-chloro-5-formyl-1*H*-imidazol-1-yl)methyl)phenylboronic acid or 3-(4-boronobenzyl)-2-ethoxy-3*H*-benzo[d]imidazole-4-carboxylic acid or (*S*)-2-(*N*-(4-boronobenzyl)pentanamido)-3-methylbutanoic acid in the preparation of angiotenzin II antagonists. The aspect of the invention is also the use of the salts and protected forms of above compounds.

In additional aspect the invention is a process where the compound prepared as described above or the compound obtainable as described above, but prepared by another process, is reacted with the compound of formula 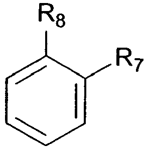 where R₈ is a halogen and R₇ is selected from the group consisting of an optionally protected tetrazole, cyano and carboxy group, to prepare an optionally substituted imidazole derivative of formula 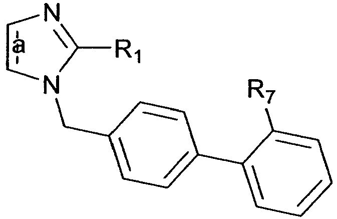 where a can be either single or double bond and said substituted imidazole is selected from any one of those of formulas: 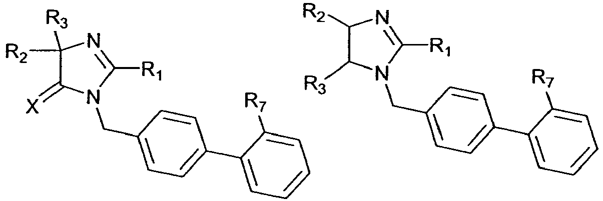 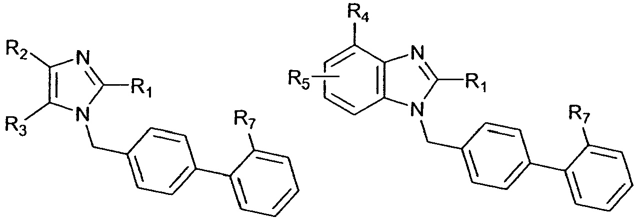 where R₁,R₂,R₃,R₄,R₅, and X and R₇ are as defined above. In an aspect of the invention any of the protecting groups are (if desired) subsequently removed and/or the obtained compound is subsequently converted into angotension II antagonist selected form the group consisting of irbesartan, telmisartan, losartan, candesartan, olmesartan, its salts and esters.

A further aspect of the invention is also a process for preparing a pharmaceutical composition comprising preparing a compound as described above, preferably preparing an angiotenzin II antagonist prepared from the above mentioned intermediate and/or its use in preparation of a medicament and/or use for manufacturing of a medicament for treating hypertension.

A specific embodiment of the invention is a process for the preparation of 2-butyl-3-[[2'-[1-(triphenylmethyl)-1*H*-tetrazol-5-yl][1,1'-biphenyl]-4-yl]methyl]-1,3-diazaspiro[4.4]non-1-en-4-one, which proceeds via novel intermediate, 4-[(2-butyl-4-oxo-1,3-diazaspiro[4.4]non-1-en-3-yl)methyl]phenylboronic acid which is prepared from 2-butyl-1,3-diazaspiro[4.4]non-1-en-4-one and 4-bromomethylphenyl boronic acid in DMF, in the presence of lithium bis(trimethylsilyl)amide at room temperature.

### Detailed Description of Invention

Our invention provides for synthesis of complicated heterocyclic system. In the synthetic scheme the catalyst enter into the synthesis in the late part when biphenyl moiety is constructed. Starting materials which enter into the biphenyl construction could be made from simple materials like 4-bromotoluene and 1-phenyltetrazole. Surprisingly the substance of the invention can be prepared from simple compounds by a short two step synthesis.

In the first step methylphenylboronic acid derivative is coupled with an imidazole derivative, preferably 2-butyl-1,3 diazaspiro[4.4]non-1-en-4-one or its analogue.

In the second step thus formed methylphenylboronic acid derivative is reacted with benzene derivative using conditions of Suzuki coupling, in the presence of palladium or nickel catalyst.

In a specific example of the process of our invention is depicted on Scheme 1.

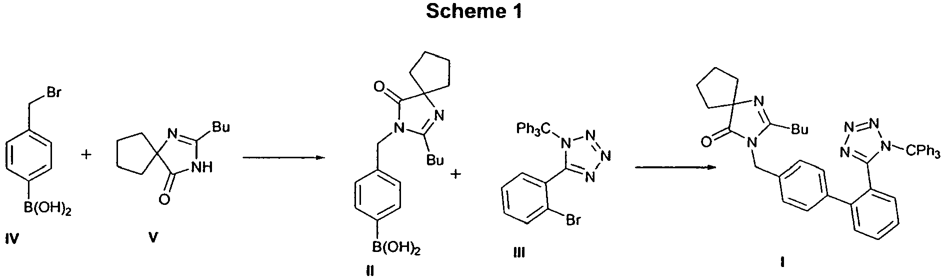

In accordance with our invention the methylphenylboronic acid derivative of the first step is presented with the formula below, 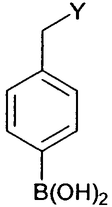 where Y represents leaving group, suitable for substitution with amino group, and is selected from the groups consisting of halogens, sulfonate esters such as tosyl, mesyl, brosyl, triflyl; phosphate of phosphite esters, or chlorosulfites, preferably Y is F, Cl, Br, I, tosyl, mesyl, brosyl or triflyl, more preferably bromo.

The preferable starting compound is thus 4-bromomethylphenyl boronic acid, which can be prepared according to the literature procedure (Snyder, H. R.; Reedy, A. J.; Lennarz, W. J. Amer. Chem. Soc. 1958, 80, 835).

The imidazole derivative 2-butyl-1,3-diazaspiro[4.4]non-1-en-4-one, or its analog if other angiotensin II antagonists besides irbesartan is being prepared, may enter reaction as a hydrochloride salt, or free base, which is prepared by neutralization using suitable base, preferably potassium of sodium hydroxide.

The imidazole derivative or its analog may be substituted imidazole, dihydroimidazole or benzimidazole and can be a compound selected from the group consisting of : 2-butyl-1,3-diazaspiro[4.4]non-1-en-4-one, 1,4'-dimethyl-2'-propyl-2,6'-bi(1*H-*benzo[d]imidazole); 2-butyl-5-chloro-1*H*-imidazole-4-carbaldehyde; (2-butyl-5-chloro-1*H*-imidazol-4-yl)methanol; 4-(2-hydroxypropan-2-yl)-2-propyl-1*H*-imidazole-5-carboxylic acid or it's protected derivatives. The imidazole derivative or its analog may be in from of salt or acid addition salt thereof, preferably hydrohalide most preferably hydrochloride salt.

The suitable imidazole analogs can be presented with general formula 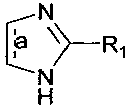 where a can be either single or double bond and can be further substituted, in preferred embodiment said imidazole is selected from any one of those of formulas, formulas below: 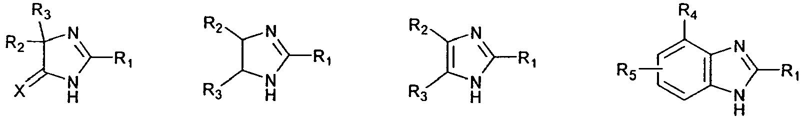 in which:
R₁ is a hydrogen, a C₁-C₆ alkyl which is unsubstituted or substituted by one or more halogen atoms, a C₂-C₆ alkenyl, a C₃-C₇ cycloalkyl, a phenyl, a phenylalkyl in which the alkyl is C₁-C₃, or a phenylalkenyl in which the alkenyl is C₂-C₃, said phenyl groups being substituted or polysubstituted by a halogen atom, a C₁-C₄ alkyl, a C₁-C₄ halogenoalkyl, C₁-C₄ polyhalogenoalkyl, a hydroxyl or a C₁-C₄ alkoxy group,
R₂ and R₃ are same or different and are each independently hydrogen or a group selected from halogen atoms, a C₁-C₆ alkyl which is unsubstituted or substituted by one or more hydroxyl groups or halogen atoms, a C₃-C₇ cycloalkyl, a C₁-C₄ alkoxy, a hydroxyl, an amino, an amino alkyl, a carboxyl, carboxyaldehide, an alkoxycarbonyl in which the alkoxy is C₁-C₄, a cyano, a tetrazolyl,
or R₂ or R₃ form a group of formula -(CH₂)ₙ-O-CO-R₆ where n is an integer between 2 and 6; and R₆ is selected from group consisted of a C₁-C₆ alkyl which is unsubstituted or substituted by one or more halogen, nitro, cyano, hydroxyl or alkoxy group; phenyl which may be unsubstituted or substituted with (nitrooxy)methyl, alkoxymethyl, or halogen atom,
or R₂ and R₃ together form a group of the formula -(CH₂)ₙ-, where n is an integer between 2 and 11,
X is an oxygen or sulphur atom,
R₄ is hydrogen, or a group selected from halogen atoms, a C₁-C₆ alkyl which is unsubstituted or substituted by one or more halogen atoms, a C₃-C₇ cycloalkyl, a C₁-C₄ alkoxy,
R₅ represents a carboxylic group, benzimidazol-2-yl or 4,5,6,7-tetrahydro-benzimidazol-2-yl group, optionally substituted in the 1-position by C₁-C₆ alkyl or a cycloalkyl group, whilst the phenyl nucleus of one of the abovementioned benzimidazole groups may additionally be substituted by a fluorine atom or by methyl or trifluoromethyl group.

The following compounds selected from the above group of imidazole derivatives are especially preferred: 2-butyl-1,3-diazaspiro[4.4]non-1-en-4-one; 1,4'-dimethyl-2'-propyl-2,6'-bi(1*H*-benzo[d]imidazole); 2-butyl-5-chloro-1*H*-imidazole-4-carbaldehyde; (2-butyl-5-chloro-1*H*-imidazol-4-yl)methanol; 4-(2-hydroxypropan-2-yl)-2-propyl-1*H-*imidazole-5-carboxylic acid or it's protected derivatives.

Reaction is performed in the solvent selected from aprotic solvents such as *N*,*N-*dimethylformamide, *N*,*N*-dimethylacetamide, acetonitrile, tetrahydrofuran, dioxan, 1-methylpyrrolidinone, 1,3-dimethyl-3,4,5,6-tetrahydro-2(1*H*)-pyrimidone, dichloromethane, dimethylsulfoxyde, toluene, benzene, or alcohols containing from one to six carbon atoms, or mixtures thereof; preferably *N*,*N*-dimethylformamide or *N*,*N*-dimethylacetamide, more preferably in *N*,*N*-dimethylformamide.

Reaction is performed in the presence of the base, selected from a group of organic bases such as lithium bis(trimethylsilyl)amide, sodium bis(trimethylsilyl)amide, hexamethyldisilazane, secondary amine, tertiary amine, sodium methoxyde, potassium methoxyde, sodium ethoxyde, potassium ethoxyde, or from a group of inorganic bases such as sodium hydride, sodium hydroxide, potassium hydroxide; preferable lithium bis(trimethylsilyl)amide or sodium hydride.

Temperature at which reaction is performed should be in range from -80 to 160°C, preferably from -10 to 25°C, more preferably at room temperature.

Preferable embodiment comprises a coupling of 2-butyl-1,3-diazaspiro[4.4]non-1-en-4-one and 4-bromomethylphenylboronic acid in *N*,*N*-dimethylformamide, in the presence of lithium bis(trimethylsilyl)amide at 25°C for up to 48 hours, preferably up to 24 hours. Another preferable embodiment comprises a coupling of 2-butyl-1,3-diazaspiro[4.4]non-1-en-4-one hydrochloride and 4-bromomethylphenylboronic acid in *N*,*N*-dimethylformamide, in the presence of sodium hydride at room temperature for up to 24 hours, preferably up to 4 hours.

Another preferred embodiment comprises a coupling of 2'-propyl-1,4'-dimethyl-2,6'-bi(1*H*-benzo[d]imidazole) (**V**) and 4-bromomethylphenylboronic acid in *N*,*N-*dimethylformamide, in the presence of lithium bis(trimethylsilyl)amide at 25°C (Scheme 2).

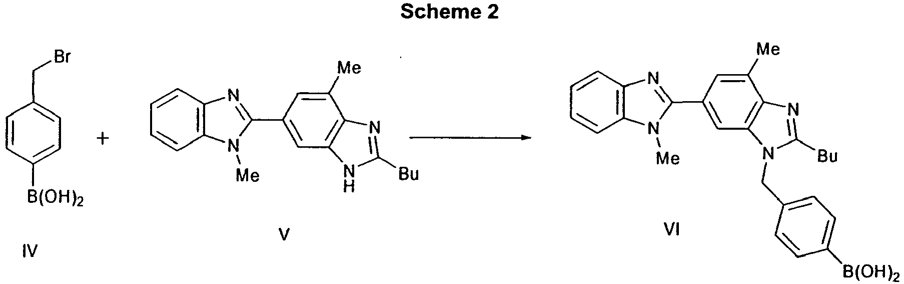

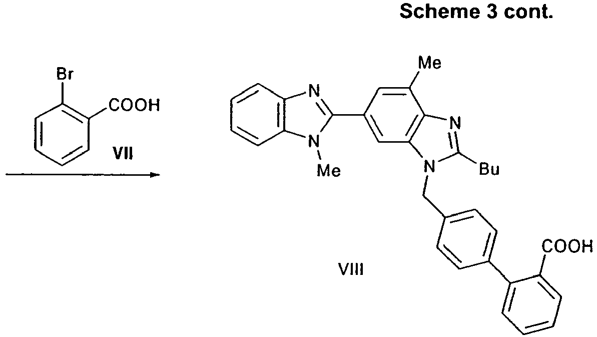

In the second step the compound obtained in first step is coupled with ortho substituted benzene derivative of formula 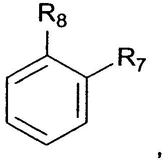 where one substituent (R₇) is a halogen, preferably bromo and the other (R₈) is an optionally protected tetrazole, cyano or carboxy group, or even a group which can be converted to tetrazole or carboxy, using conditions of Suzuki coupling, in the presence of palladium or nickel catalyst.

In a specific embodiment 4-[(2-butyl-4-oxo-1,3-diazaspiro[4.4]non-1-en-3-yl)methyl]phenylboronic acid (**II**) or its analog and 5-(2-bromophenyl)-1-(triphenylmethyl)-1 H-tetrazole (**III**) are coupled using conditions of Suzuki coupling, in the presence of palladium or nickel catalyst.

The catalyst may be selected from the group consisting of palladium complexes and nickel complexes.

Preferable catalyst comprises a combination of palladium complex and triaryl phosphine. More preferable, catalyst is tetrakis(triphenylphosphine)palladium(0), neat or prepared *in situ* from palladium (**II**) acetate and triphenyl phosphine. Most preferably the catalyst is tetrakis(triphenylphosphine)palladium(0).

Reaction is performed in organic solvent selected from aprotic solvents such as *N*,*N-*dimethylformamide, *N*,*N*-dimethylacetamide, acetonitrile, tetrahydrofuran, dioxan, 1-methylpyrrolidinone, 1,3-dimethyl-3,4,5,6-tetrahydro-2(1*H*)-pyrimidone, dichloromethane, dimethylsulfoxyde, toluene, benzene, or alcohols containing from one to six carbon atoms, or mixtures thereof, preferably in mixture of toluene, ethanol and water.

Reaction is performed in the presence of aqueous solution of sodium or potassium carbonate. Reaction is performed in the temperature interval from -80°C to 160°C, preferably from 25 to 120°C, more preferably at the reflux temperature of the mixture. Preferably the reaction is performed in the mixture of toluene, ethanol and water

An embodiment represents the coupling of 4-[(2-butyl-4-oxo-1,3-diazaspiro[4.4]non-1-en-3-yl)methyl]phenylboronic acid_(**II**) and 5-(2-bromophenyl)-1-(triphenylmethyl)-1*H*-tetrazole **(III),** in the presence of tetrakis(triphenylphosphine)palladium(0) and sodium carbonate. Preferably the reaction proceeds in mixture of degassed toluene and aqueous solution of sodium carbonate for several hours, preferably up to 24 hours, more preferably up to 6 hours at reflux temperature.

Another embodiment represents the coupling of 4'-[(1,4'-dimethyl-2'-propyl-[2,6'-bi-1*H*-benzimidazol]-1'-yl)methyl]phenylboronic acid (**VI**) and 2-bromobenzoic acid (**VII**) in the mixture of toluene, methanol and water, in the presence of tetrakis(triphenylphosphine)palladium(0) and sodium carbonate, at reflux temperature of the reaction mixture.

The following examples are offered to illustrate aspects of the present invention, and are not intended to limit or define the present invention in any manner.

### Example 1 Preparation of 4-[(2-Butyl-4-oxo-1,3-diazaspiro[4.4]non-1-en-3-yl) methyl]phenylboronic acid (compound of formula II)

To a solution of 3.37g of 2-butyl-1,3-diazaspiro[4.4]non-1-en-4-on hydrochloride in the 50 ml mixture of acetone/water 1:1, a solution of 0.58 g NaOH in 10 ml water was added. Then 40 ml water was added and extracted twice with 50 ml CH₂Cl₂. Combined organic phases were dried over anhydrous magnesium sulphate and concentrated, then dissolved in 30 ml DMF, cooled to 0°C and stirred under inert atmosphere. 15 ml of 1M solution LiN(SiMe₃)₂ in THF was slowly added, and stirred at 0°C for additional 20 min, then solution of 1.05 g compound of formula IV in 40 ml DMF was slowly added to the reaction mixture, which was then left to warm up to r.t. and stirred at r.t for 20-24h. After that, 200 ml of ethyl acetate was added in washed twice with brine. Organic phase was dried over anhydrous magnesium sulphate, and then concentrated. After addition of diethyl ether, white precipitate was collected by filtration to give compound of formula II (1.93 g).
MS, (M+H)⁺= 329 m/z. M.p.=107-134°C
¹H NMR (300MHz, DMSO-d₆): δ 0.76 (t, J=5.2, 3H), 1.22 (m, 2H), 1.44 (m, 2H), 1.65 (m, 2H), 1.82 (m, 6H), 2.28 (t, J=7.6, 2H), 4.66 (s, 2H), 7.08 (d, 8.0, 2H), 7.74 (d, J=8.0, 2H), 8.05 (s, 2H).
¹³C NMR (300MHz, DMSO-d₆): δ 13.6, 21.5, 25.4 (2), 26.6, 27,5, 36.8 (2), 42.6, 75.8, 125.4 (2), 134.5 (2), 138.9, 161.1, 185.7.

### Example 2 Preparation of 4-[(2-Butyl-4-oxo-1,3-diazaspiro[4.4]non-1-en-3-yl) methyl]phenylboronic acid (compound of formula II)

Suspension of 1.065 g of 2-butyl-1,3-diazaspiro[4.4]non-1-en-4-on hydrochloride in 25 ml DMF was cooled to 0°C, then 0.462 g of sodium hydride (60% suspension in mineral oil) was added and left to warm to r.t, then stirred at this temperature for two hours. Reaction mixture was then cooled to 0°C, and solution of 1.15 g of compound of formula IV in 10 ml DMF was slowly added. After allowing reaction mixture to warm up, it was stirred at r.t for 3-4 hours, and then 100 ml od ethyl acetate was added, and washed twice with brine. Organic phase was dried over magnesium sulphate, concentrated, and then after addition of diethyl ether, white precipitate was collected by filtration to give compound of formula II (0.89g).

### Example 3 Preparation of 2-butyl-3-[[2'-[1-(triphenylmethyl)-1H-tetrazol-5-yl][1,1'-biphenyl]-4-yl]methyl]-1,3-diazaspiro[4.4]non-1-en-4-one (compound of formula I)

The mixture of 463 mg of 5-(2-bromofenil)-1-(triphenylmethyl)-1H-tetrazole (formula III) and 55 mg tetrakis(triphenylphosphine)palladium(0) was dissolved in 25 ml of degassed toluene, and stirred for 15 min under inert atmosphere. A solution of 235 mg sodium carbonate in 3 ml of water was added, followed by solution of 322 mg of compound of formula II in 8 ml of ethanol. The mixture was then refluxed under inert atmosphere for 5-6h then cooled to r.t. Afterwards, 100 ml of dichloromethane was added, then organic phase passed through pad of silica, dried over anhydrous magnesium sulphate and concentrated. After addition of diethyl ether, trityl irbesartane (compound of formula I) crystallizes from reaction mixture (0.47g).
MS, (M+H)⁺=671 m/z
¹H NMR (300MHz, DMSO-d₆):δ 0.70 (t, J=7.3, 3H), 1.12 (m, 2H), 1.41 (m, 2H), 1.67 (m, 2H), 1,83 (m, 6H), 2,19 (t, J=7.5, 2H), 4.62 (s, 2H), 6.86 (t, J=7.3, 6H), 7.04 (m, 4H), 7.32 (m, 10H), 7.53 (m, 2H), 7.78 (d, J=7.7, 1 H).
¹³C NMR (300MHz, DMSO-d₆): δ 13.6, 21.4, 25.5 (2), 26.4, 27.4, 36.8 (2), 42.2, 75.8, 82.3, 125.7, 126.1 (2), 127.8 (6), 128.3 (3), 129.3 (2), 129.6 (6), 130.3, 130.4, 130.6, 135.8, 139.4, 140.8 (3), 141.1

### Example 4 Preparation of 4'-[(1,4'-dimethyl-2'-propyl-[2,6'-bi-1H-benzimidazol]-1'-yl)methyl]phenylboronic acid (compound of formula VI)

A solution of 1.32g 2'-propyl-1,4'-dimethyl-2,6'-bi(1H-benzo[d]imidazole) (**V**) in DMF (40 ml), under inert atmosphere (N₂) is cooled (0°C), then 4.33 ml of 1 M solution LiN(SiMe₃)₂ in THF was slowly added, stirred for another 30 min, then slowly solution of 0.93 g compound of formula IV in 15 ml DMF was added to the reaction mixture, which was then left to warm up to r.t. and stirred at r.t for 20-24h. After that, 200 ml of ethyl acetate was added in washed twice with brine. Organic phase was dried over anhydrous magnesium sulphate, and then concentrated. After addition of diethyl ether, light brown precipitate was collected by filtration to give compound of formula VI (0.94g).
MS, (M+H)⁺=439 m/z
¹H NMR (300MHz, DMSO-d₆): δ 0.95 (t, J=7.5, 3H), 1.76 (m, 2H), 2.62 (s, 3H), 2.86 (t, J=7.8, 2H), 3.81 (s, 3H), 5.57 (s, 2H), 7.08 (d, J=8.1, 2H), 7.23 (m, 2H), 7.46 (s, 1 H), 7.60 (m, 2H), 7.70 (s, 1 H), 7.72 (d, J=8.1, 2H), 8.02 (s, 2H).

## Claims

1. A process for reacting a compound of formula: 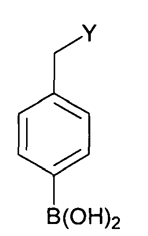 where Y represents leaving group which is selected from the groups consisting of halogens, sulfonate esters; phosphate of phosphite esters, and chlorosulfites, with optionally substituted imidazole of formula 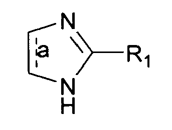 where a can be either single or double bond and said imidazole is selected from any one of those of formulas, 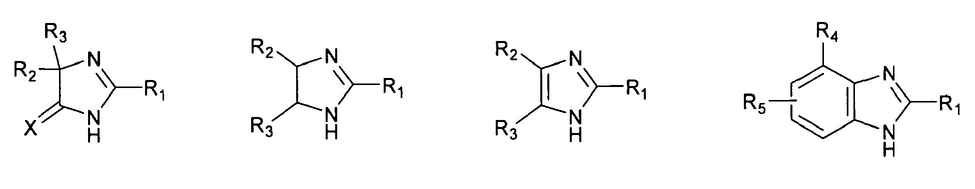 in which:
R₁ is a hydrogen, a C₁-C₆ alkyl optionally substituted by one or more halogen atoms, a C₂-C₆ alkenyl, a C₃-C₇ cycloalkyl, a phenyl, a phenylalkyl in which the alkyl is C₁-C₃, or a phenylalkenyl in which the alkenyl is C₂-C₃, where said phenyl group is optionally substituted by one or mors substituent selected from halogen atom, a C₁-C₄ alkyl, a C₁-C₄ halogenoalkyl, C₁-C₄ polyhalogenoalkyl, a hydroxyl or a C₁-C₄ alkoxy group,
R₂ and R₃ are same or different and each independently represent hydrogen or a group selected from halogen, a C₁-C₆ alkyl which is unsubstituted or substituted by one or more hydroxyl groups or halogen atoms, a C₃-C₇ cycloalkyl, a C₁-C₄ alkoxy, a hydroxyl, an amino, an amino alkyl, a carboxyl, carboxyaldehide, an alkoxycarbonyl in which the alkoxy is C₁-C₄, a cyano, a tetrazolyl,
or R₂ or R₃ form a group of formula -(CH₂)ₙ-O-CO-R₆ where n is an integer between 2 and 6; and R₆ is selected from group consisted of a C₁-C₆ alkyl which is unsubstituted or substituted by one or more halogen, nitro, cyano, hydroxyl or alkoxy group; phenyl which is unsubstituted or substituted with (nitrooxy)methyl, alkoxymethyl, or halogen atom,
or R₂ and R₃ together form a group of the formula -(CH₂)ₙ-, where n is an integer between 2 and 11,
X is an oxygen or sulphur atom,
R₄ is a hydrogen, or a group selected from halogen atoms, a C₁-C₆ alkyl which is unsubstituted or substituted by one or more halogen atoms, a C₃-C₇ cycloalkyl, a C₁-C₄ alkoxy,
R₅ represents a carboxylic group, benzimidazol-2-yl or 4,5,6,7-tetrahydro-benzimidazol-2-yl group, optionally substituted in the 1-position by C₁-C₆ alkyl or a cycloalkyl group, whilst the phenyl nucleus of one of the abovementioned benzimidazole groups may additionally be substituted by a fluorine atom or by methyl or trifluoromethyl group,
or their corresponding hydrohalides
where a compounds presented with any of the formulae 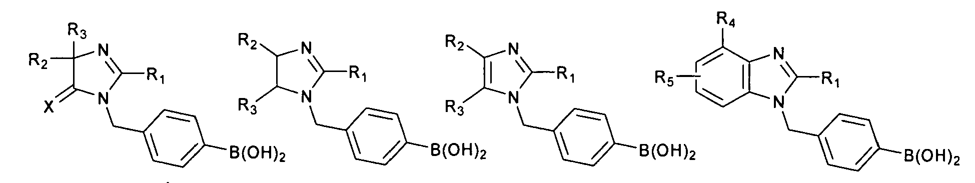 are prepared.

2. A process according to the previous claim where Y is selected from the groups consisting of Br, Cl, F, I, tosyl, mesyl, brosyl and triflyl.

3. A process according to any one of previous claims, where imidazole is selected from the group consisting of is 2-butyl-1,3-diazaspiro[4.4]non-1-en-4-on, 1,4'-dimethyl-2'-propyl-2,6'-bi(1H-benzo[d]imidazole); 2-butyl-5-chloro-1 H-imidazole-4-carbaldehyde; (2-butyl-5-chloro-1H-imidazol-4-yl)methanol; 4-(2-hydroxypropan-2-yl)-2-propyl-1 H-imidazole-5-carboxylic acid or salt of any of those.

4. A process according to any one of previous claims, where one of the reactants is 4-bromomethylphenylboronic acid.

5. A process according to any one of previous claims, where reaction is performed in organic solvent selected from aprotic solvents selected from the group consisting of *N*,*N*-dimethylformamide, *N*,*N*-dimethylacetamide, acetonitrile, tetrahydrofuran, dioxan, 1-methylpyrrolidinone, 1,3-dimethyl-3,4,5,6-tetrahydro-2(1*H*)-pyrimidone, dichloromethane, dimethylsulfoxyde, toluene, benzene, alcohols containing from one to six carbon atoms, and mixtures thereof.

6. A process according to any one of previous claims, where reaction is performed in the presence of a base.

7. A process according to the previous claim where the base is an organic base selected from the group consisting of lithium bis(trimethylsilyl)amide, sodium bis(trimethylsilyl)amide, hexamethyldisilazane, secondary amine, tertiary amine, sodium methoxyde, potassium methoxyde, sodium ethoxyde, and potassium ethoxyde.

8. A process according to any one of claims 1 to 5, where reaction is performed in a presence of an inorganic base selected from the group consisting of sodium hydride, sodium hydroxide and potassium hydroxide.

9. A process according to any one of previous claims, where reaction is performed in the temperature range from -80°C to 160°C.

10. A compound selected from the group consisting of 4-[(2-butyl-4-oxo-1,3-diazaspiro[4.4]non-1-en-3-yl)methyl]phenylboronic acid, 4'-[(1,4'-dimethyl-2'-propyl-[2,6'-bi-1*H*-benzimidazol]-1'-yl)methyl]phenylb oronic acid, 1-(4-boronobenzyl)-4-(2-hydroxypropan-2-yl)-2-propyl-1*H*-imidazole-5-carboxylic acid, 4-((2-butyl-4-chloro-5-(hydroxymethyl)-1*H*-imidazol-1-yl)methyl)phenylboronic acid, 4-((2-butyl-4-chloro-5-formyl-1*H*-imidazol-1-yl)methyl)phenylboronic acid, 3-(4-boronobenzyl)-2-ethoxy-3*H*-benzo[d]imidazole-4-carboxylic acid, and (*S*)-2-(*N*-(4-boronobenzyl)pentanamido)-3-methylbutanoic acid.

11. Use of a compound selected from the group consisting of 4-[(2-butyl-4-oxo-1,3-diazaspiro[4.4]non-1-en-3-yl)methyl]phenylboronic acid, 4'-[(1,4'-dimethyl-2'-propyl-[2,6'-bi-1*H*-benzimidazol]-1'-yl)methyl]phenylboronic acid, 1-(4-boronobenzyl)-4-(2-hydroxypropan-2-yl)-2-propyl-1*H*-imidazole-5-carboxylic acid, 4-((2-butyl-4-chloro-5-(hydroxymethyl)-1*H*-imidazol-1-yl)methyl)phenylboronic acid, 4-((2-butyl-4-chloro-5-formyl-1*H*-imidazol-1-yl)methyl)phenylboronic acid, 3-(4-boronobenzyl)-2-ethoxy-3*H-*benzo[*d*]imidazole-4-carboxylic acid and (*S*)-2-(*N*-(4-boronobenzyl)pentanamido)-3-methylbutanoic acid in the preparation of angiotensin II antagonists.

12. A process where the compound obtained according to any of any of the claims 1 to 9 is reacted with the compound of formula 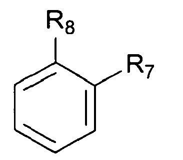 where R₈ is a halogen and R₇ is selected from the group consisting of an optionally protected tetrazole, cyano and carboxy group, to prepare an optionally substituted imidazole derivative of formula 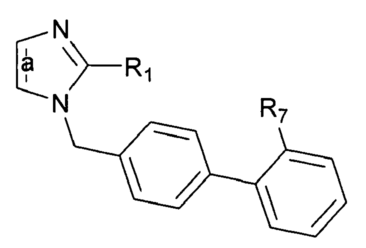 where a can be either single or double bond and said imidazole is selected from any one of those of formulas, 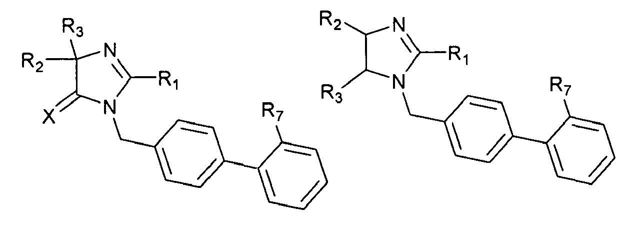 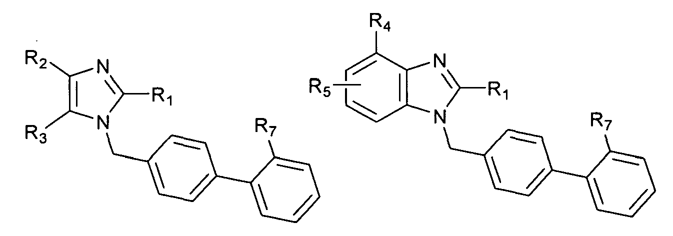 and R₁,R₂,R₃,R₄,R₅, and X are as defined in claim 1 and R₇ as defined above.

13. A process where the compound prepared according to any of the claims 1 to 9 is subsequently reacted with a compound of formula 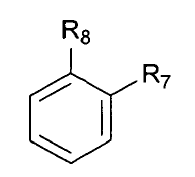 where R₈ is a halogen and R₇ is selected from the group consisting of an optionally protected tetrazole, optionally protected carboxy group and cyano group, to prepare an optionally substituted imidazole derivative of formula 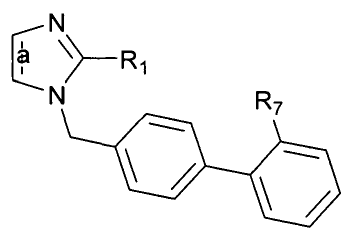 where a can be either single or double bond and said imidazole is selected from any one of those of formulas, 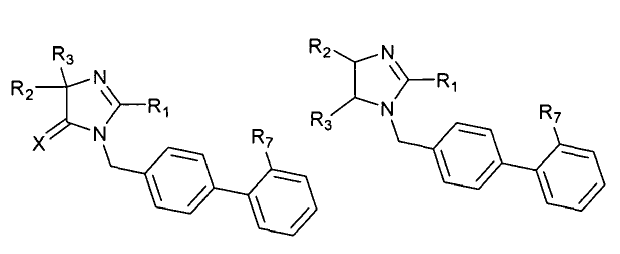 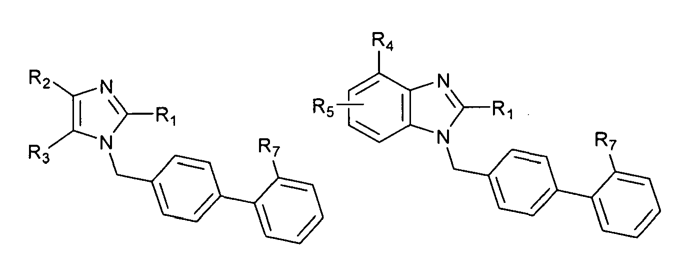 and R₁,R₂,R₃,R₄,R₅, and X are as defined in claim 1 and R₇ as defined above.

14. A process for preparing 2-butyl-3-[[2'-[1-(triphenylmethyl)-1*H*-tetrazol-5-yl][1,1'-biphenyl]-4-yl]methyl]-1,3-diazaspiro[4.4]non-1-en-4-one, comprising the step of reacting 4-[(2-butyl-4-oxo-1,3-diazaspiro[4.4]non-1-en-3-yl)methyl]phenylboronic acid with 5-(2-bromofenil)-1-(triphenylmethyl)-1*H*-tetrazole.

15. A process according to any of the claims 12 to 14, **characterized in that** a catalyst selected from the group consisting of palladium complexes and nickel complexes is used.

16. A process according to previous claim, wherein the catalyst comprises a combination of a palladium complex and a triaryl phosphine.

17. A process according to any of the claims 12 to 16, where reaction is performed in a solvent where the solvent comprises one or more of the solvents selected from the group consisting of *N*,*N*-dimethylformamide, *N*,*N*-dimethylacetamide, acetonitrile. tetrahydrofuran, dioxan, 1-methylpyrrolidinone, 1,3-dimethyl-3,4,5,6-tetrahydro-2(1*H*)-pyrimidone, dichloromethane, dimethylsulfoxyde, toluene, benzene, alcohols containing from one to six carbon atoms and water.

18. A process according to any one of claims 12 to 17 **characterized in that** it is performed in the presence of inorganic base selected from potassium carbonate or sodium carbonate.

19. A process according to any one of claims 12 to 18, where reaction is performed in the temperature range from -80°C to 160°C.

20. A process according to previous claim, where reaction is performed at the reflux temperature of the mixture of the solvents.

21. A process according to any of claims 12 to 20 comprising a subsequent step in which any of the protecting groups are removed.

22. A process according to any of claims 12 to 21, wherein said process is carried out and the obtained compound is subsequently converted into angotensin II antagonist selected from the group consisting of irbesartan, telmisartan, losartan, candesartan. olmesartan, its salts and esters.

23. A process for preparing a pharmaceutical composition consisting of preparing a compound by carrying out a process as defined in any one of the claims 12 to 22.

## Patentansprüche

1. Verfahren zum Reagieren lassen einer Verbindung der Formel: 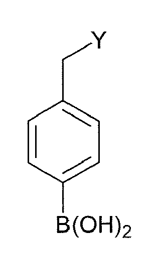 wobei Y eine Abgangsgruppe darstellt, welche ausgewählt ist aus der Gruppe bestehend aus Halogenen, Sulfonatestern, Phosphat von Phosphitestern, und Chlorsulfiten, mit optional substituiertem Imidazol der Formel 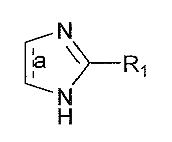 wobei a entweder eine Einzel- oder Doppelbindung sein kann und das Imidazol ausgewählt ist aus irgendeiner dieser Formeln 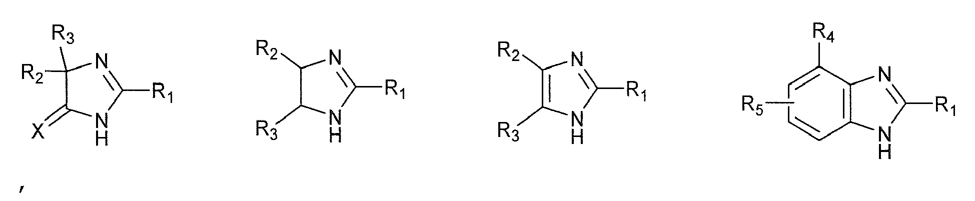 in welchen:
R₁ ein Wasserstoff, ein C₁-C₆-Alkyl, optional substituiert mit einem oder mehreren Halogenatomen, ein C₂-C₆-Alkenyl, ein C₃-C₇-Cycloalkyl, ein Phenyl, ein Phenylalkyl, in welchem das Alkyl C₁-C₃ ist, oder ein Phenylalkenyl, in welchem das Alkenyl C₂-C₃ ist, wobei diese Phenylgruppe optional substituiert ist durch einen oder mehrere Substituenten ausgewählt aus Halogenatom, einem C₁-C₄-Alkyl, einem C₁-C₄ - Halogenalkyl, C₁-C₄-Polyhalogenalkyl, einem Hydroxyl oder einer C₁-C₄-Alkoxygruppe ist;
R₂ und R₃ gleich oder verschieden sind, und jeder unabhängig Wasserstoff oder eine Gruppe ausgewählt aus Halogen, einem C₁-C₆-Alkyl, welches unsubstituiert oder substituiert ist durch eine oder mehrere Hydroxylgruppen oder Halogenatome, einem C₃-C₇-Cycloalkyl, einem C₁-C₄-Alkoxy, einem Hydroxyl, einem Amino, einem Aminoalkyl, einem Carboxyl, Carboxyaldehyd, einem Alkoxycarbonyl in welchem das Alkoxy C₁-C₄ ist, einem Cyano oder einem Tetrazolyl darstellt, oder R₂ oder R₃ eine Gruppe der Formel -(CH₂)n-O-CO-R₆ bilden, wobei n eine ganze Zahl von 2 bis 6 ist; und R₆ ausgewählt ist aus der Gruppe bestehend aus einem C₁-C₆-Alkyl, welches unsubstituiert oder unsubstituiert ist mit einer oder mehreren Halogen-, Nitro-, Cyano-, Hydroxyl- oder AlkoxyGruppen; Phenyl welches unsubstituiert oder substituiert ist mit (Nitrooxy)methyl, Alkoxymethyl, oder Halogenatom, oder R₂ und R₃ zusammen eine Gruppe der Formel -(CH₂)ₙ-bilden, wobei n eine ganze Zahl von 2 bis 11 ist,
X ein Sauerstoff- oder Schwefelatom ist,
R₄ ein Wasserstoff, oder eine Gruppe ausgewählt aus Halogenatomen, einem C₁-C₆-Alkyl welches unsubstituiert oder substituiert ist mit ein oder mehreren Halogenatomen, einem C₃-C₇-Cycloalkyl, einem C₁-C₄-Alkoxy, ist,
R₅ eine Carboxyl-Gruppe, Benzimidazol-2-yl- oder eine 4,5,6,7-Tetrahydro-benzimidazol-2-yl-Gruppe, optional substituiert in der 1-Position durch C₁-C₆-Alkyl oder einer Cycloalkyl-Gruppe, darstellt, während der Phenyl-Kern von einer der oben erwähnten Benzimidazol-Gruppen zusätzlich durch ein Fluoratom oder durch Methyl- oder TrifluormethylGruppe substituiert sein kann,
oder deren entsprechenden Hydrohalogeniden,
wobei Verbindungen, dargestellt durch irgendeine der Formeln 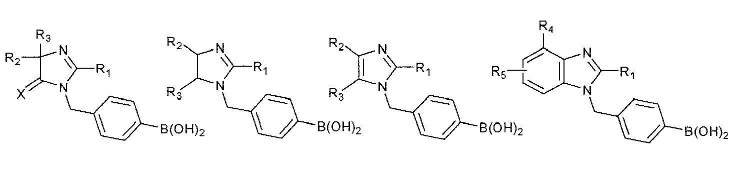 hergestellt werden.

2. Verfahren gemäß dem vorhergehenden Anspruch, wobei Y ausgewählt ist aus den Gruppen bestehend aus Br, Cl, F, I, Tosyl, Mesyl, Brosyl und Triflyl.

3. Verfahren gemäß irgendeinem der vorhergehenden Ansprüche, wobei Imidazol ausgewählt ist aus der Gruppe bestehend aus 2-Butyl-1,3-diazaspiro[4,4]non-1-en-4-on, 1,4'-Dimethyl-2'-propyl-2,6'-bi(1H-benzo[d]imidazol); 2-Butyl-5-chlor-1H-imidazol-4-carbaldehyd; (2-Butyl-5-chlor-1H-imidazol-4-yl)methanol; 4-(2-Hydroxypropan-2-yl)-2-propyl-1H-imidazol-5-carbonsäure oder Salz von irgendeinem von diesen.

4. Verfahren gemäß irgendeinem der vorhergehenden Ansprüche, wobei einer der Reaktanden 4-Brommethylphenylborsäure ist.

5. Verfahren gemäß irgendeinem der vorhergehenden Ansprüche, wobei die Reaktion in organischem Lösungsmittel durchgeführt wird, ausgewählt aus aprotischen Lösungsmitteln ausgewählt aus der Gruppe bestehend aus *N*,*N*-Dimethylformamid, *N*,*N*-Dimethylacetamid, Acetonitril, Tetrahydrofuran, Dioxan, 1-Methylpyrrolidinon, 1,3-Dimethyl-3,4,5,6-tetrahydro-2(1*H*)-pyrimidon, Dichlormethan, Dimethylsulfoxid, Toluol, Benzol, Alkoholen enthaltend von einem bis sechs Kohlenstoffatomen, und Mischungen davon.

6. Verfahren gemäß irgendeinem der vorhergehenden Ansprüche, wobei die Reaktion in Gegenwart einer Base durchgeführt wird.

7. Verfahren gemäß dem vorhergehenden Anspruch, wobei die Base eine organische Base ist, ausgewählt aus der Gruppe bestehend aus Lithium bis(trimethylsilyl)amid, Natrium bis(trimethylsilyl)amid, Hexamethyldisilazan, sekundäres Amin, tertiäres Amin, Natriummethoxid, Kaliummethoxid, Natriumethoxid, und Kaliumethoxid.

8. Verfahren gemäß irgendeinem der Ansprüche 1 bis 5, wobei die Reaktion durchgeführt wird in der Gegenwart einer anorganischen Base, ausgewählt aus der Gruppe bestehend aus Natriumhydrid, Natriumhydroxid und Kaliumhydroxid.

9. Verfahren gemäß irgendeinem der vorhergehenden Ansprüche, wobei die Reaktion in dem Temperaturbereich von -80°C bis 160°C durchgeführt wird.

10. Verbindung, ausgewählt aus der Gruppe bestehend aus
4-[(2-Butyl-4-oxo-1,3-diazaspiro[4,4]non-1-en-3-yl)methyl]phenylborsäure, 4'-[(1,4'-Dimethyl-2'-propyl-[2,6'-bi-1*H*-benzimidazol]-1'-yl)methyl]phenylborsäure,
1-(4-Boronobenzyl)-4-(2-hydroxypropan-2-yl)-2-propyl-1*H-*imidazol-5-carbonsäure,
4-((2-Butyl-4-chlor-5-(hydroxymethyl)-1*H*-imidazol-1-yl)methyl)phenylborsäure,
4-((2-Butyl-4-chlor-5-formyl-1*H*-imidazol-1-yl)methyl)phenylborsäure,
3-(4-Boronobenzyl)-2-ethoxy-3*H*-benzo[d]imidazol-4-carbonsäure, und
(*S*)-2-(*N*-(4-Boronobenzyl)pentanamido)-3-methylbutansäure.

11. Verwendung einer Verbindung, ausgewählt aus der Gruppe bestehend aus
4-[(2-Butyl-4-oxo-1,3-diazaspiro[4,4]non-1-en-3-yl)methyl]-phenylborsäure,
4'-[(1,4'-Dimethyl-2'-propyl-[2,6'-bi-1*H*-benzimidazol]-1'-yl)methyl]phenylborsäure,
1-(4-Boronobenzyl)-4-(2-hydroxypropan-2-yl)-2-propyl-1*H-*imidazol-5-carbonsäure,
4-((2-Butyl-4-chlor-5-(hydroxymethyl)-1*H*-imidazol-1-yl)methyl)phenylborsäure,
4-((2-Butyl-4-chlor-5-formyl-1*H*-imidazol-1-yl)methyl)phenylborsäure,
3-(4-Boronobenzyl)-2-ethoxy-3*H*-benzo[d]imidazol-4-carbonsäure und
(*S*)-2-(*N*-(4-Boronobenzyl)pentanamido)-3-methylbuttersäure bei der Herstellung von Angiotensin II Antagonisten.

12. Verfahren, worin die Verbindung, erhalten gemäß irgendeinem der Ansprüche 1 bis 9, mit der Verbindung der Formel 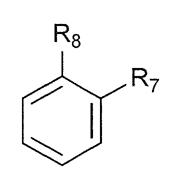 zur Reaktion gebracht wird,
wobei R₈ ein Halogen ist und R₇ ausgewählt ist aus der Gruppe bestehend aus einem optional geschützten Tetrazol, Cyano- und Carboxy-Gruppe, um ein optional substituiertes Imidazol-Derivat der Formel 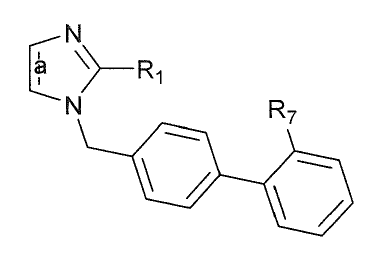 herzustellen,
wobei a entweder eine Einzel- oder Doppelbindung sein kann und das Imidazol ausgewählt ist aus irgendeiner von diesen Formeln 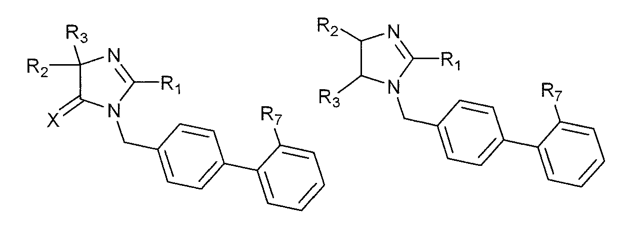 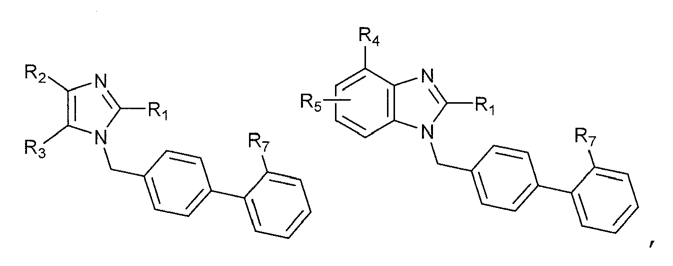 und R₁, R₂, R₃, R₄, R₅ und X wie in Anspruch 1 definiert sind und R₇ wie oben definiert ist.

13. Verfahren, worin die gemäß irgendeinem der Ansprüche 1 bis 9 hergestellte Verbindung anschließend reagiert wird mit einer Verbindung der Formel 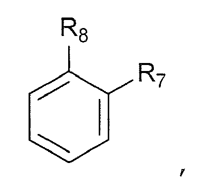 wobei R₈ ein Halogen ist und R₇ ausgewählt ist aus der Gruppe bestehend aus einem optional geschützten Tetrazol, optional geschützten Carboxy-Gruppe und Cyano-Gruppe, um ein optional substituiertes Imidazol-Derivat der Formel 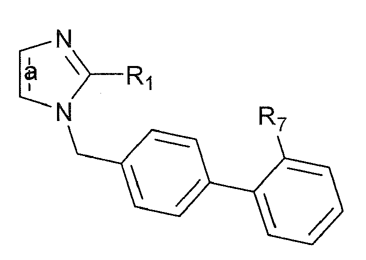 herzustellen,
wobei a entweder eine Einzel- oder Doppelbindung sein kann und das Imidazol ausgewählt ist aus irgendeiner von diesen Formeln 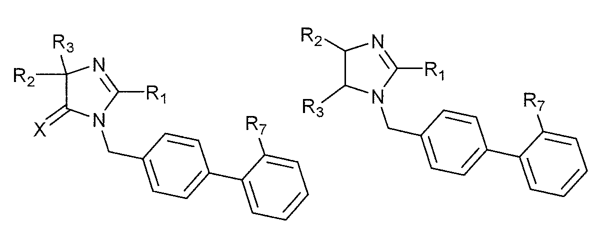 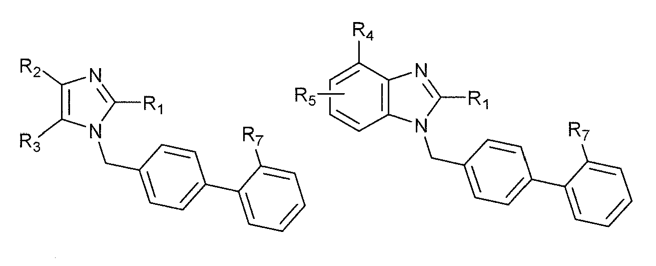 und R₁,R₂,R₃,R₄,R₅ und X wie in Anspruch 1 definiert sind und R₇ wie oben definiert ist.

14. Verfahren zur Herstellung von 2-Butyl-3-[[2'-[1-(triphenylmethyl)-1*H*-tetrazol-5-yl][1,1'-biphenyl]-4-yl]methyl]-1,3-diazaspiro[4,4]non-1-en-4-on, umfassend den Schritt des Reagieren lassens von 4-[(2-butyl-4-oxo-1,3-diazaspiro[4,4]non-1-en-3-yl)methyl]phenylborsäure mit 5-(2-Bromfenil)-1-(triphenylmethyl)-1*H*-tetrazol.

15. Verfahren gemäß irgendeinem der Ansprüche 12 bis 14, **dadurch gekennzeichnet, dass** ein Katalysator ausgewählt aus der Gruppe bestehend aus Palladium-Komplexen und Nickel-Komplexen verwendet wird.

16. Verfahren gemäß dem vorhergehenden Anspruch, wobei der Katalysator eine Kombination aus einem Palladium-Komplex und einem Triarylphosphin umfasst.

17. Verfahren gemäß irgendeinem der Ansprüche 12 bis 16, wobei die Reaktion in einem Lösungsmittel durchgeführt wird, wobei das Lösungsmittel eines oder mehrere der Lösungsmittel ausgewählt aus der Gruppe bestehend aus *N*,*N*-Dimethylformamid, N,N-Dimethylacetamid, Acetonitril, Tetrahydrofuran, Dioxan, 1-Methylpyrrolidinon, 1,3-Dimethyl-3,4,5,6-tetrahydro-2(1*H*)-pyrimidon, Dichlormethan, Dimethylsulfoxid, Toluol, Benzol, Alkoholen enthaltend von einem bis sechs Kohlenstoffatome, und Wasser, umfasst.

18. Verfahren gemäß irgendeinem der Ansprüche 12 bis 17, **dadurch gekennzeichnet, dass** es in der Gegenwart einer anorganischen Base, ausgewählt aus Kaliumcarbonat oder Natriumcarbonat durchgeführt wird.

19. Verfahren gemäß irgendeinem der Ansprüche 12 bis 18, wobei die Reaktion in dem Temperaturbereich von -80°C bis 160°C durchgeführt wird.

20. Verfahren gemäß dem vorhergehenden Anspruch, wobei die Reaktion bei der Rückfluss-Temperatur der Mischung der Lösungsmittel durchgeführt wird.

21. Verfahren gemäß irgendeinem der Ansprüche 12 bis 20, umfassend einen nachfolgenden Schritt, in welchem irgendeine der Schutzgruppen entfernt wird.

22. Verfahren gemäß irgendeinem der Ansprüche 12 bis 21, wobei dieses Verfahren durchgeführt wird und die erhaltene Verbindung anschließend in Angiotensin II Antagonist ausgewählt aus der Gruppe bestehend aus Irbesartan, Telmisartan, Losartan, Candesartan, Olmesartan, ihre Salze und Ester, umgewandelt wird.

23. Verfahren zur Herstellung einer pharmazeutischen Zusammensetzung, bestehend aus dem Herstellen einer Verbindung durch Durchführen eines Verfahrens wie in irgendeinem der Ansprüche 12 bis 22 definiert.

## Revendications

1. Procédé de mise en réaction d'un composé de formule : 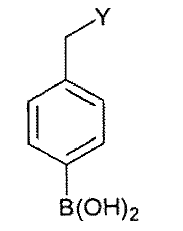 dans laquelle Y représente un groupe sortant qui est sélectionné parmi le groupe constitué par des halogènes, des esters de sulfonate ; du phosphate d'esters de phosphite, et des chlorosulfites, avec de l'imidazole éventuellement substitué, de formule : 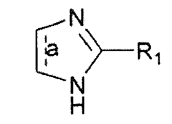 dans laquelle a peut être soit une simple, soit une double liaison et ledit imidazole est sélectionné parmi l'une quelconque de ces formules : 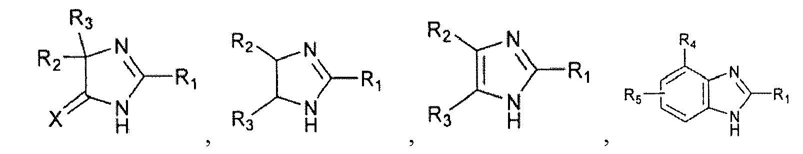 dans lesquelles :
R₁ représente de l'hydrogène, un alkyle en C₁-C₆ éventuellement substitué par un ou plusieurs atomes d'halogène, un alkényle en C₂-C₆, un cycloalkyle en C₃-C₇, un phényle, un phénylalkyle dans lequel l'alkyle est en C₁-C₃, ou un phénylalkényle dans lequel l'alkényle est en C₂-C₃, dans lequel ledit groupe phényle est éventuellement substitué par un ou plusieurs substituants sélectionnés parmi un atome d'halogène, un alkyle en C₁-C₄, un halogénoalkyle en C₁-C₄, un polyhalogénoalkyle en C₁-C₄, un hydroxyle ou un groupe alkoxy en C₁-C₄,
R₂ et R₃ sont identiques ou différents, et chacun représente indépendamment l'hydrogène ou un groupe sélectionné parmi un halogène, un alkyle en C₁-C₆ qui est non substitué ou substitué par un ou plusieurs groupes hydroxyles ou atomes d'halogène, un cycloalkyle en C₃-C₇, un alkoxy en C₁-C₄, un hydroxyle, un amino, un amino-alkyle, un carboxyle, un carboxyaldéhide, un alkoxycarbonyle dans lequel l'alkoxy est en C₁-C₄, un cyano, un tétrazolyle,
ou R₂ ou R₃ forment un groupe de formule -(CH₂)ₙ-O-CO-R₆ dans lequel n est un nombre entier entre 2 et 6 ; et R₆ est sélectionné parmi le groupe constitué par un alkyle en C₁-C₆ qui est non substitué ou substitué par un ou plusieurs groupes halogène, nitro, cyano, hydroxyle ou alkoxy ; un phényle qui est non substitué ou substitué avec du (nitrooxy)méthyle, de l'alkoxyméthyle, ou un atome d'halogène,
ou R₂ et R₃ forment ensemble un groupe de formule -(CH₂)ₙ-, dans lequel n est un nombre entier entre 2 et 11,
X est un atome d'oxygène ou de soufre,
R₄ représente de l'hydrogène, ou un groupe sélectionné parmi des atomes d'halogène, un alkyle en C₁-C₆ qui est non substitué ou substitué par un ou plusieurs atomes d'halogène, un cycloalkyle en C₃-C₇, un alkoxy en C₁-C₄,
R₅ représente un groupe carboxylique , un groupe benzimidazol-2-yl ou 4,5,6,7-tétrahydro-benzimidazol-2-yl, éventuellement substitué en position 1 par un alkyle en C₁-C₆ ou un groupe cycloalkyle, alors que le noyau phényle de l'un des groupes benzimidazole mentionnés ci-dessus peut en outre être substitué par un atome de fluor ou par un groupe méthyle ou trifluorométhyle,
ou leurs hydrohalides correspondants,
dans lequel des composés présentant l'une quelconque des formules : 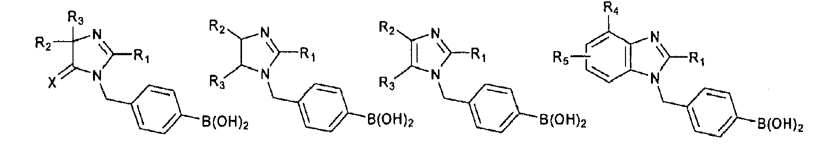 sont préparés.

2. Procédé selon la revendication précédente, dans lequel Y est sélectionné parmi le groupe constitué par les atomes de Br, Cl, F, I, le tosyle, le mésyle, le brosyle et le triflyle.

3. Procédé selon l'une quelconque des revendications précédentes, dans lequel l'imidazole est sélectionné parmi le groupe constitué par le 2-butyl-1,3-diazaspiro[4.4]non-1-en-4-on, le 1,4'-diméthyl-2'-propyl-2,6'-bi(1H-benzo[d]imidazole) ; le 2-butyl-5-chloro-1H-imidazole-4-carbaldéhyde ; le (2-butyl-5-chloro-1H-imidazol-4-yl)méthanol ; l'acide 4-(2-hydroxypropan-2-yl)-2-propyl-1H-imidazole-5-carboxylique ou un sel de l'un quelconque de ceux-ci.

4. Procédé selon l'une quelconque des revendications précédentes, dans lequel l'un des réactifs est l'acide 4-bromométhylphénylboronique.

5. Procédé selon l'une quelconque des revendications précédentes, dans lequel la réaction est mise en oeuvre dans un solvant organique sélectionné parmi des solvants aprotiques sélectionnés parmi le groupe constitué par le N,N-diméthylformamide, le N,N-diméthylacétamide, l'acétonitrile, le tétrahydrofurane, le dioxane, le 1-méthylpyrrolidinone, le 1,3-diméthyl-3,4,5,6-tétrahydro-2(1H)-pyrimidone, le dichlorométhane, le diméthylsulfoxyde, le toluène, le benzène, des alcools contenant de un à six atomes de carbone, et leurs mélanges.

6. Procédé selon l'une quelconque des revendications précédentes, dans lequel la réaction est mise en oeuvre en présence d'une base.

7. Procédé selon la revendication précédente dans lequel la base est une base organique sélectionnée parmi le groupe constitué par le bis(triméthylsilyl)amide de lithium, le bis(triméthylsilyl)amide de sodium, l'hexaméthyldisilazane, une amine secondaire, une amine tertiaire, le méthoxyde de sodium, le méthoxyde de potassium, l'éthoxyde de sodium, et l'éthoxyde de potassium.

8. Procédé selon l'une quelconque des revendications 1 à 5, dans lequel la réaction est mise en oeuvre en présence d'une base inorganique sélectionnée parmi le groupe constitué par l'hydrure de sodium, l'hydroxyde de sodium et l'hydroxyde de potassium.

9. Procédé selon l'une quelconque des revendications précédentes, dans lequel la réaction est mise en oeuvre dans une plage de température de -80°C à 160°C.

10. Composé sélectionné parmi le groupe constitué par :
l'acide 4-[(2-butyl-4-oxo-1,3-diazaspiro [4.4]non-1-en-3-yl)méthyl]phénylboronique, l'acide 4'-[(1,4'-diméthyl-2'-propyl-[2,6'-bi-1H-benzimidazol]-1'-yl)méthyl]phénylboronique, l'acide 1-(4-boronobenzyl)-4-(2-hydroxypropan-2-yl)-2-propyl-1H-imidazole-5-carboxylique, l'acide 4-((2-butyl-4-chloro-5-(hydroxyméthyl)-1H-imidazol-1-yl)méthyl)phénylboronique, l'acide 4-((2-butyl-4-chloro-5-formyl-1H-imidazol-1-yl)méthyl)phénylboronique, l'acide 3-(4-boronobenzyl)-2-éthoxy-3H-benzo[d]imidazole-4-carboxylique, et l'acide (S)-2-(N-(4-boronobenzyl)pentanamido)-3-méthylbutanoïque.

11. Utilisation d'un composé sélectionné parmi le groupe constitué par l'acide 4-[(2-butyl-4-oxo-1,3-diazaspiro[4.4]non-1-en-3-yl)méthyl]phénylboronique, l'acide 4'-[(1,4'-diméthyl-2'-propyl-[2,6'-bi-1H-benzimidazol]-1'-yl)méthyl]phénylboronique, l'acide 1-(4-boronobenzyl)-4-(2-hydroxypropan-2-yl)-2-propyl-1H-imidazole-5-carboxylique, l'acide 4-((2-butyl-4-chloro-5-(hydroxyméthyl)-1H-imidazol-1-yl)méthyl)phénylboronique, l'acide 4-((2-butyl-4-chloro-5-formyl-1H-imidazol-1-yl)méthyl)phénylboronique, l'acide 3-(4-boronobenzyl)-2-éthoxy-3H-benzo[d]imidazole-4-carboxylique, et l'acide (S)-2-(N-(4-boronobenzyl)pentanamido)-3-méthylbutanoïque lors de la préparation d'antagonistes de l'angiotensine II.

12. Procédé dans lequel le composé obtenu selon l'une quelconque des revendications 1 à 9 est mis en réaction avec le composé de formule : 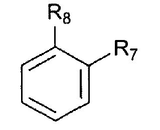 dans laquelle R₈ est un halogène et R₇ est sélectionné parmi le groupe constitué par un groupe tétrazole, cyano et carboxy éventuellement protégé, afin de préparer un dérivé d'imidazole éventuellement substitué de formule : 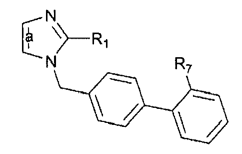 dans lequel a peut être soit une simple soit une double liaison et ledit imidazole est sélectionné parmi l'une quelconque de ces formules : 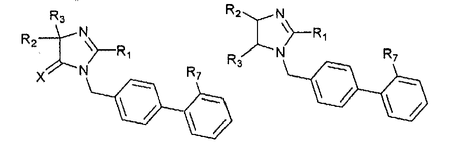 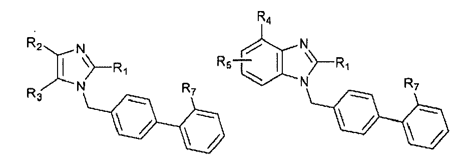 et R₁, R₂, R₃, R₄, R₅, et X sont tels que définis dans la revendication 1 et R₇ est tel que défini précédemment.

13. Procédé dans lequel le composé préparé selon l'une quelconque des revendications 1 à 9 est ensuite mis en réaction avec un composé de formule 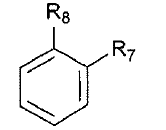 dans laquelle R₈ est un halogène et R₇ est sélectionné parmi le groupe constitué par un groupe tétrazole éventuellement protégé, carboxy éventuellement protégé et un groupe cyano, afin de préparer un dérivé d'imidazole éventuellement substitué de formule : 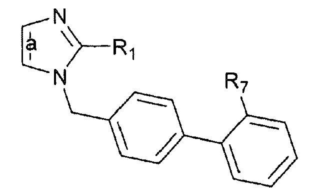 dans lequel a peut être soit une simple soit une double liaison et ledit imidazole est
sélectionné parmi l'une quelconque des formules suivantes, 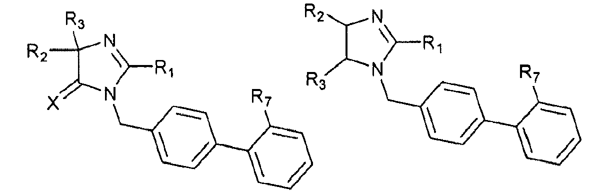 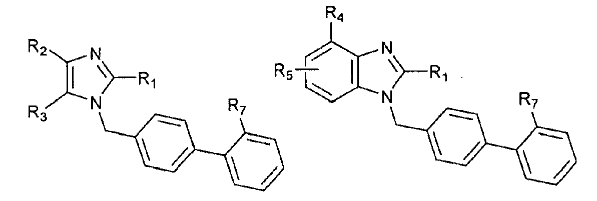 et R₁, R₂, R₃, R₄, R₅, et X sont tels que définis dans la revendication 1 et R₇ est tel que défini précédemment.

14. Procédé de préparation de 2-butyl-3-[[2'-[1-(triphénylméthyl)-1H-tétrazol-5-yl] [1,1'-biphényl]-4-yl]méthyl]-1,3-diazaspiro[4.4]non-1-en-4-one, comprenant l'étape de mise en réaction d'acide 4-[(2-butyl-4-oxo-1,3-diazaspiro[4.4]non-1-en-3-yl)méthyl]phénylboronique avec du 5-(2-bromophényl)-1-(triphénylméthyl)-1H-tétrazole.

15. Procédé selon l'une quelconque des revendications 12 à 14, **caractérisé en ce qu'**un catalyseur sélectionné parmi le groupe constitué par des complexes de palladium et des complexes de nickel est utilisé.

16. Procédé selon la revendication précédente, dans lequel le catalyseur comprend un mélange d'un complexe de palladium et d'une phosphine triaryle.

17. Procédé selon l'une quelconque des revendications 12 à 16, dans lequel la réaction est mise en oeuvre dans un solvant dans lequel le solvant comprend un ou plusieurs des solvants sélectionnés parmi le groupe constitué par le N,N-diméthylformamide, le N,N-diméthylacétamide, l'acétonitrile, le tétrahydrofurane, le dioxane, le 1-méthylpyrrolidinone, le 1,3-diméthyl-3,4,5,6-tétrahydro-2(1H)-pyrimidone, le dichlorométhane, le diméthylsulfoxyde, le toluène, le benzène, des alcools contenant de un à six atomes de carbone et l'eau.

18. Procédé selon l'une quelconque des revendications 12 à 17 **caractérisé en ce qu'**il est mis en oeuvre en la présence d'une base inorganique sélectionnée à partir du carbonate de potassium ou du carbonate de sodium.

19. Procédé selon l'une quelconque des revendications 12 à 18, dans lequel la réaction est mise en oeuvre dans la plage de température de -80°C à 160°C.

20. Procédé selon la revendication précédente, dans lequel la réaction est mise en oeuvre à la température de reflux du mélange des solvants.

21. Procédé selon l'une quelconque des revendications 12 à 20 comprenant une étape consécutive dans laquelle tous les groupes de protection sont enlevés.

22. Procédé selon l'une quelconque des revendications 12 à 21, dans lequel ledit procédé est mis en oeuvre et le composé obtenu est transformé consécutivement en un antagoniste de l'angiotensine II sélectionné parmi le groupe constitué par l'irbésartan, le telmisartan, le losartan, le candésartan, l'olmésartan, leurs sels et esters.

23. Procédé de préparation d'une composition pharmaceutique consistant à préparer un composé en mettant en oeuvre un procédé selon l'une quelconque des revendications 12 à 22.
